# EUROPEAN PATENT SPECIFICATION

(11) **EP 2 784 474 B1**
(45) Date of publication and mention of the grant of the patent: **22.06.2016**
(21) Application number: 14159473.9
(22) Date of filing: 13.03.2014
(51) Int. Cl.: G01N 1/31, G01N 35/00, C12Q 1/68, G01N 15/14

(54) **Cell analyzing method**
Zellanalyseverfahren
Procédé d'analyse de cellules

(30) Priority: 29.03.2013 JP 2013071041; 22.01.2014 JP 2014009537
(43) Date of publication of application: 01.10.2014
(73) Proprietor: SYSMEX CORPORATION, Kobe-shi, Hyogo 651-0073 (JP)
(72) Inventor: Morita, Masakatsu, Hyogo, 651-0073 (JP); Oguro, Masahiko, Hyogo, 651-0073 (JP); Yokoyama, Koji, Hyogo, 651-0073 (JP); Yamamoto, Yuka, Hyogo, 651-0073 (JP)
(74) Representative: Hoffmann Eitle

(56) References cited:
- US-A1- 2001 043 884
- US-A1- 2009 111 101
- US-A1- 2012 214 195
- Hanane Derradji ET AL: "Comparison of different protocols for telomere length estimation by combination of quantitative fluorescence in situ hybridization (Q-FISH) and flow cytometry in human cancer cell lines", Anticancer research, 1 March 2005 (2005-03-01), page 1039, XP055131106, Greece Retrieved from the Internet: URL:http://ar.iiarjournals.org/content/25/ 2A/1039.abstract [retrieved on 2014-07-24]
- Geoffrey Rolls: "Fixation and Fixatives (5) - Practical procedures to optimise quality, the effects of heat and microwaves", , 6 March 2012 (2012-03-06), XP055131177, Wetzlar, Germany Retrieved from the Internet: URL:http://www.leicabiosystems.com/patholo gyleaders/fixation-and-fixatives-5-practic al-procedures-to-optimise-quality-the-effe cts-of-heat-and-microwaves/ [retrieved on 2014-07-23]

## Description

### FIELD OF THE INVENTION

The present invention relates to a cell analyzing method employed to analyze cells collected from living organisms.

### BACKGROUND

Conventionally, there have been known a cell analyzer configured to automatically analyze the DNA content of cells and provide canceration information of the cells based on the analyzed DNA content.

For example, US2009/091746 discloses a cell analyzer, wherein cells collected from a test subject are subjected to treatments, such as PI staining, to prepare a measurement sample, the prepared measurement sample is flown into a flow cell and light is irradiated on the measurement sample flowing in the flow cell to obtain fluorescent light signals and scattered light signals of the respective cells, and waveforms of the obtained signals are analyzed to extract characteristic parameters that reflect thereon such information as DNA content, nucleus size, and cell size. The cell analyzer then determines whether the cells are cancerous or dysplastic based on the characteristic parameters.

It is disclosed in WO2012/039097 that a biosample container containing an alcohol-containing medium where the cells are preserved is set in a cell analyzer, the medium in the biosample container is substituted with a diluent and subjected to treatments, such as PI staining, in the cell analyzer to prepare a measurement sample to be flown in a flow cell.

Derradji H. et al. Anticancer Research 25: 1039-1050 (2005) entitled "Comparison of Different Protocols for Telomere Length Estimation by Combination of Quantitative Fluorescence In situ Hybridization (Q-FISH) and Flow Cytometry in Human Cancer Cell Lines*"* discloses a method for analysing a cell, comprising regulating a temperature of a medium, wherein the cell is preserved and obtaining optical information by irradiating light on the cell. The cells are grown in RPMI medium at 37°C and then fixed in a medium comprising alcohol at room temperature.

### SUMMARY OF THE INVENTION

The scope of the present invention is defined solely by the appended claims, and is not affected to any degree by the statements within this summary.

According to the disclosure of WO2012/039097, the cells to be analyzed are preserved in the alcohol-containing medium. When the cells are thus preserved in the medium as disclosed in the document, however, the preservation temperature of the medium containing the cells differs from a hospital or a test facility to another. In some cases, the medium are preserved at room temperature, or in another cases, they are preserved in a refrigerator. When the medium containing the cells is transported from a hospital to a test facility or the like, it is possible that the preservation temperature changes depending on environments where the medium is transported. Thus, the preservation temperature of the medium is variable according to different conditions of preservation and transportation.

The inventors of the present invention used a medium where cells were preserved in such a cell analyzer as disclosed in WO2012/039097 to analyze the cells. The inventors learned from this analysis that there was variability in measured optical information (information of fluorescent and scattered lights) due to different preservation temperatures of the medium.

The present invention was accomplished to solve the problems of these related arts. An object of the present invention is to provide a cell analyzing method that can control such variability of optical information due to different preservation temperatures of a medium where cells are preserved.

In the wake of continued studies and researches on the relationship between the preservation temperature of the medium where the cells are preserved and the optical information obtained by the cell analyzer, the inventors of the present invention found the way of controlling such variability of the optical information obtained from the cells preserved in the medium fed to the cell analyzer; the variability can be controlled when the temperature of the medium containing the cells and fed to the cell analyzer is regulated to 35°C to 45°C. Based on this finding, the inventors finally accomplished the present invention.

A method for analyzing a cell according to the present invention comprises: regulating a temperature of a medium comprising alcohol, wherein the cell is fixed by the medium; staining DNA of the alcohol-fixed cell with a fluorescent staining solution containing a dye; flowing the cell into a flow cytometer; and obtaining a fluorescent light information by irradiating light from a light source of the flow cytometer on the cell, wherein the method is characterized in that the temperature of the medium is regulated to 35°C to 45°C.

The cell analyzing method provided by the present invention includes the step of regulating the temperature of the medium to 35°C to 45°C. According to this step, variability of the optical information obtained in the measuring step can be suitably controlled irrespective of the preservation temperature of the medium. Therefore, any processes thereafter executed based on the optical information can obtain accurate results unaffected by the preservation temperature of the medium.

An example of a cell analyzer not part of the present invention comprises: a temperature regulating unit configured to regulate a temperature of a medium to a predetermined degree, wherein the cell is preserved in the medium; a measurement sample preparing device configured to prepare a measurement sample by substituting the medium with a diluent; and a measuring device configured to obtain optical information by irradiating light on the cell included in the measurement sample.

This exemplary cell analyzer includes the temperature regulating unit configured to regulate the temperature of the medium to a predetermined degree. According to this configuration, variability of the optical information obtained by the measuring device can be suitably controlled irrespective of the preservation temperature of the medium fed to the cell analyzer. Therefore, any processes thereafter executed based on the optical information can obtain accurate results unaffected by the preservation temperature of the medium.

The present invention succeeds in controlling variability of optical information due to difference in preservation temperatures of a medium where cells are preserved.

### BRIEF DESCRIPTION OF THE DRAWINGS

Fig. 1 is a perspective view of an overall structure of a cell analyzer according to an example;
Fig.2 schematically shows the internal configuration of a measuring device provided in the cell analyzer;
Fig. 3 shows the configuration of a flow cytometer;
Fig. 4 is a flow chart showing steps of an operation performed by the cell analyzer;
Fig. 5A is a diagram of a forward scattered light signal (FSC) and a side fluorescent light signal (SFL) obtained in a measuring operation; Fig. 5B is a schematic enlarged view in cross section of epithelial cells in uterine cervix; Fig. 5C is a graph showing a relationship between an N/C ratio and cell size;
Fig. 6A is a diagram showing a relationship between DNA content and cell count in a cell cycle; Fig. 6B is a diagram of DNA content that changes per cell cycle;
Fig. 7 is a scattergram showing a relationship between an N/C ratio and cell width (cell size);
Fig. 8 is a histogram showing a relationship between DNA content and cell count;
Fig. 9 is a graph showing the result of a test 1-1 on epithelial cells in uterine cervix;
Fig. 10 is a graph showing the result of a test 1-2 on epithelial cells in uterine cervix;
Fig. 11 is a graph showing the result of a test 1-3 on epithelial cells in uterine cervix;
Fig. 12 is a graph showing the result of a test 1-4 on epithelial cells in uterine cervix;
Fig. 13 is a graph showing the result of a test 1-5 on epithelial cells in uterine cervix;
Fig. 14 is a graph showing the result of a test 1-6 on epithelial cells in uterine cervix;
Fig. 15 is a graph showing the result of a test 1-7 on epithelial cells in uterine cervix;
Fig. 16 is a graph showing the result of a test 2 on epithelial cells in uterine cervix;
Fig. 17 is a graph showing the result of a test 3-1 on epithelial cells in oral mucosa;
Fig. 18 is a graph showing the result of a test 3-2 on epithelial cells in oral mucosa; and
Fig. 19 is a graph showing the result of a test 3-3 on epithelial cells in oral mucosa.

### DETAILED DESCRIPTION OF THE PREFERRED EMBODIMENTS

The preferred embodiments of the present invention will be described hereinafter with reference to the drawings.

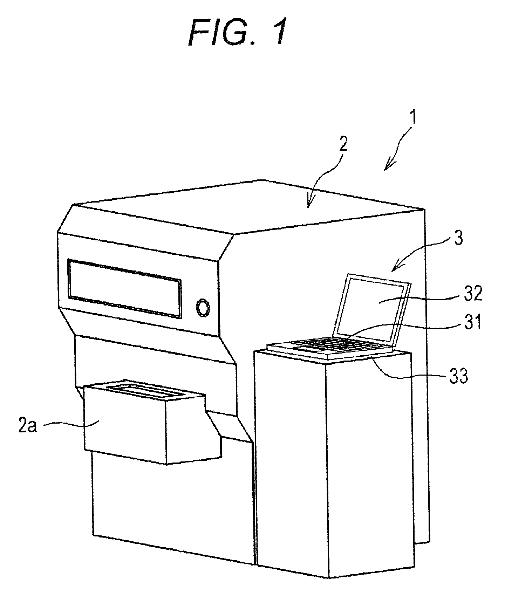

Fig. 1 is a perspective view of an overall structure of a cell analyzer.

A cell analyzer 1 according to the present example is characterized in that a measurement sample containing cells (biosample) collected from a patient (test subject) is flown into a flow cell, laser light is irradiated on the measurement sample flowing in the flow cell to detect lights from the measurement sample (forward scattered light, side scattered light, side fluorescent light), and optical signals of these lights are analyzed to determine whether the tested cells include any cancerous cells or cells that are growing into cancerous cells (hereinafter, collectively referred to as "cancerous cells"). More specifically, the cell analyzer 1 is used for screening of uterine cervical cancer, wherein epithelial cells of uterine cervix collected from patients are used.

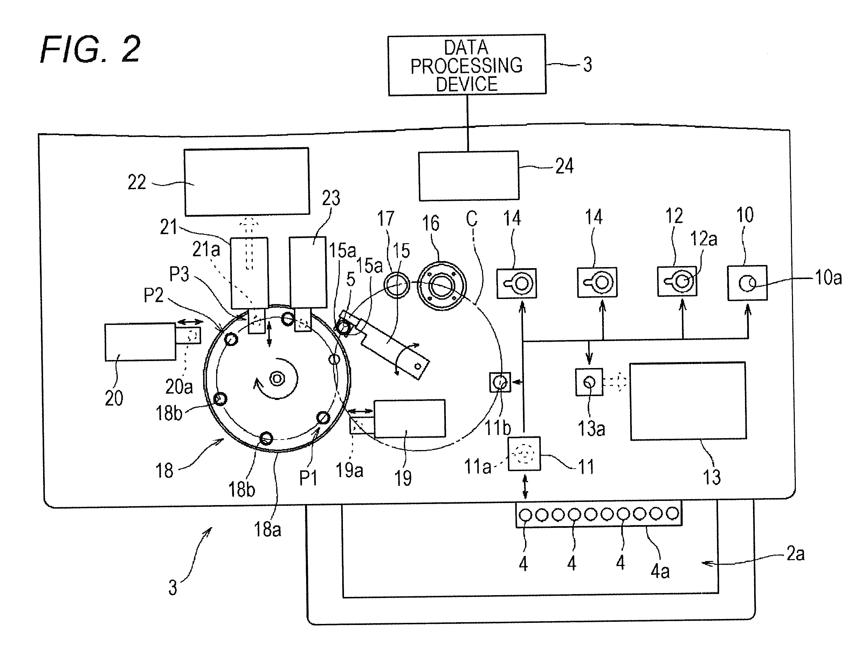

As shown in Fig. 1, the cell analyzer 1 has a measuring device 2 and a data processing device 3. The measuring device 2 performs operations such as a measuring operation of a biosample collected from a patient. The data processing device 3, connected to the measuring device 2, performs operations such as analyzing and displaying (outputting) of a result obtained from the measuring operation. An analyte setting unit 2a is installed on the front side of the measuring device 2. In the analyte setting unit 2a, there are located a plurality of sample containers 4 (see Fig. 2) each containing therein a medium consisting primarily of methanol in which the biosample (cells) collected from a patient is preserved. The data processing device 3 has an input unit 31, a display unit 32, and a controller 33.

### [Configuration of measuring device 2]

Fig. 2 schematically shows the internal configuration of the measuring device provided in the cell analyzer.

As shown in Fig. 2, the analyte setting unit 2a transports racks 4a holding a plurality of sample containers 4 one after another to a position at which the sample is suctioned by an analyte pipette unit 11.

The analyte pipette unit 11 transfers the sample in the sample container 4 to a temperature regulating unit 10. The analyte pipette unit 11 transfers the sample in the temperature regulating unit 10 to a first dispersing unit 12. The analyte pipette unit 11 transfers the sample in the first dispersing unit 12 to a sub-detecting unit 13 and a discriminating and substituting unit 14. The analyte pipette unit 11 feeds a measurement sample container 5 with a concentrated solution obtained in the discriminating and substituting unit 14. The analyte pipette unit 11 can move to positions in the upper direction of the temperature regulating unit 10, a sample container portion 12a of the first dispersing unit 12, a sample fetching portion 13a of the sub-detecting unit 13, the discriminating and substituting unit 14, and the measurement sample container 5 located in a sample delivering and receiving portion 11b.

The analyte pipette unit 11 has a pipette 11a that suctions and discharges the sample, and is configured to quantify the sample using a sample quantifier not shown in the drawing (such as quantifying cylinder, motor that drives a piston in the quantifying cylinder). The analyte pipette unit 11 is further configured to feed a predetermined amount of sample to the units and portions.

The temperature regulating unit 10 regulates the temperature of the medium transported from the sample container 4 of the analyte setting unit 2a to a predetermined degree. Specifically, the temperature regulating unit 10 has a sample setting portion 10a where the sample container to be temperature-regulated is set. The temperature regulating unit 10 is configured to warm the sample container in the sample setting portion 10a and thereby regulate the temperature of the medium in the sample container.

The first dispersing unit 12 performs a first dispersing treatment to disperse aggregated cells included in the sample. Specifically, the first dispersing treatment is a shearing force applying treatment in which a shearing force is applied to the aggregated cells to disperse them. The first dispersing unit 12 includes a sample container portion 12a that can contain the sample therein, and is configured to mechanically apply the shearing force to the aggregated cells in the sample fed to the sample container portion 12a.

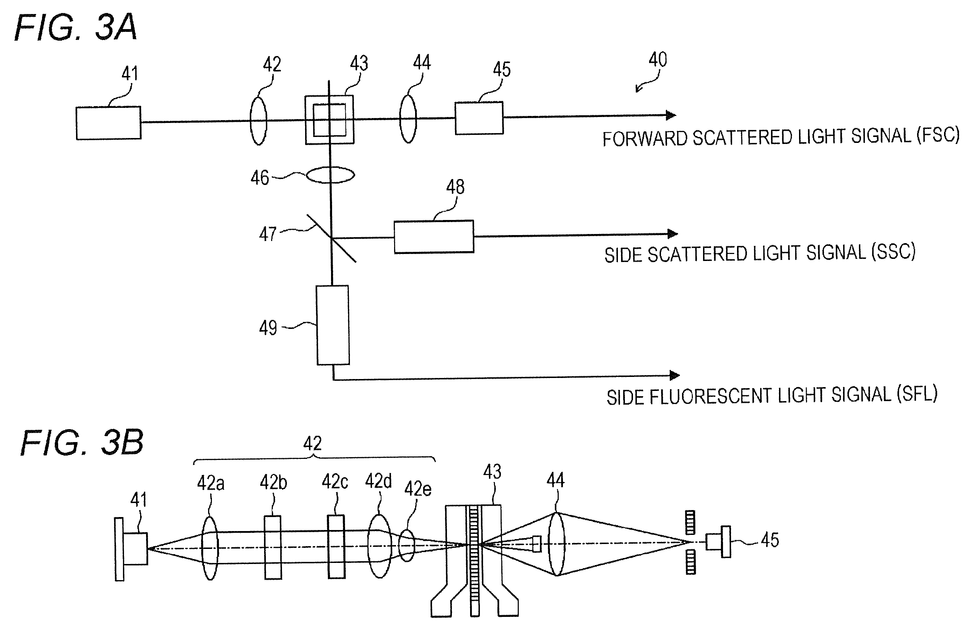

The sub-detecting unit 13 measures the concentration of the sample before the measuring operation performed by a main detecting unit 22 starts. The sub-detecting unit 13 has a flow cytometer 40 (see Fig. 3A) almost identical to a flow cytometer provided in the main detecting unit 22 which will be described later.

The discriminating and substituting unit 14 receives the sample after the first dispersing treatment is performed thereto by the first dispersing unit 12, and substitutes (dilutes) the medium of the sample consisting primarily of methanol with a diluent. Specifically, the medium of the sample consisting primarily of methanol is filtered to a predetermined extent by a filter that allows passage of fluids but not cells, and a buffer solution is added to the filtered medium. This lessens any impacts on DNA staining described later that may be caused by methanol in the medium. As a result, DNA of any cells to be measured can be favorably stained. Also, the discriminating and substituting unit 14 discriminates the cells to be measured included in the sample (epithelial cells in uterine cervix collected from a test subject) from any other cells (for example, red blood cells, white blood cells, bacteria) and impurities. Further, the discriminating and substituting unit 14 concentrates the cells to be measured included in the sample to obtain a cell count required in the measuring operation performed by the main detecting unit 22. To increase the efficiency of the operation, two discriminating and substituting units 14 are provided.

A container transport unit 15 holds the measurement sample container 5 set in a reaction unit 18 using a holding portion 15a having a scissors-like shape, and transports the measurement sample container 5 thereby held to the sample delivering and receiving portion 11b, a second dispersing unit 16, a liquid removing unit 17, and then to the reaction unit 18. The container transport unit 15 is configured to move the holding portion 15a along a predetermined circumferential trajectory. The container transport unit 15 is also configured to be able to move the holding portion 15a upward and downward. The sample delivering and receiving portion 11b, the second dispersing unit 16, the liquid removing unit 17, and the reaction unit 18 are located on the circumferential trajectory. Therefore, the measurement sample container 5 set in the reaction unit 18, when held by the holding portion 15a of the container transport unit 15, can be transported to the portions and units located on the circumferential trajectory.

The second dispersing unit 16 performs a second dispersing treatment which differs from the first dispersing treatment to the sample after the first dispersing treatment is performed thereto by the first dispersing unit 12. Specifically, after the sample is subjected to the first dispersing treatment performed by the first dispersing unit 12 and then concentrated in the discriminating and substituting unit 14 (in which cells to be measured are enriched), the second dispersing unit 16 imparts supersonic vibration to the resulting sample. The second dispersing unit 16 disperses the aggregated cells that remained after the first dispersing treatment into single cells.

After the second dispersing treatment is performed by the second dispersing unit 16, the liquid removing unit 17 removes liquid from the outer surface of the measurement sample container 5 (draining). The second dispersing treatment is performed to the measurement sample container 5 dipped in liquid. Therefore, the liquid removing unit 17 sends an air flow to the outer surface of the measurement sample container 5 to remove any liquid droplets left on the outer surface thereof. This prevents transfer of the liquid to the other structural components, such as the reaction unit, 18 when the measurement sample container 5 is set therein.

The reaction unit 18 accelerates reactions between the sample in the measurement sample container 5 and reagents added to the sample by a first reagent adding unit 19 and a second reagent adding unit 20. The reaction unit 18 has a circular rotary table 18a that can be rotated by a driving unit not shown in the drawing. A plurality of retaining portions 18b formed so as to retain the measurement sample containers 5 therein is provided in an outer peripheral part of the rotary table 18a. The measurement sample container 5 is set in the retaining portion 18b. The trajectory of the retaining portion 18b drawn by the rotation of the rotary table 18a and the circumferential trajectory of the holding portion 15a of the container transport unit 15 intersect with each other at a predetermined position. The container transport unit 15 is configured to set the measurement sample container 5 in the retaining portion 18b at the intersecting position. The reaction unit 18 warms the measurement sample container 5 set in the retaining portion 18b to a predetermined temperature (about 37°C) and thereby accelerates the reactions between the sample and the reagents.

The first reagent adding unit 19 and the second reagent adding unit 20 respectively feed the reagents into the measurement sample container 5 set in the retaining portion 18b. The first reagent adding unit 19 and the second reagent adding unit 20 are located at positions in vicinity of the outer peripheral part of the rotary table 18a, and respectively have feeders 19a and 20a that can move to positions P1 and P2 in the upper direction of the measurement sample container 5 set on the rotary table 18a. When the measurement sample container 5 is transported by the rotary table 18a to and arrives at the position P1, P2, a predetermined quantity of reagent can be fed from the feeder 19a, 20a into the measurement sample container 5.

The reagent added by the first reagent adding unit 19 is RNase used for RNA isolation from the cells. The reagent added by the second reagent adding unit 20 is a staining solution used for DNA staining of the cells. The RNA isolation decomposes RNA in a cell so that DNA of cell nucleus alone can be measured. The DNA staining uses a propidium iodide (PI) solution which is a fluorescent staining solution containing dyes. The DNA staining selectively stains the cell nucleus, enabling the detection of fluorescence from the cell nucleus. Herein, the "measurement sample preparing device" according to the example includes all of the structural components wherein the sample is prepared from the biosample that are located between the temperature regulating unit 10 and the main detecting unit 22. The "measurement sample preparing unit" according to the present embodiment includes the dispersing units 12 and 16, the discriminating and substituting unit 14, the reaction unit 18, and the reagent adding units 19 and 20.

A sample suctioning unit 21 suctions the sample (measurement sample) in the measurement sample container 5 set in the retaining portion 18b and transfers the suctioned measurement sample to the main detecting unit 22. The sample suctioning unit 21 is located at a position in vicinity of the outer peripheral part of the rotary table 18a, and has a pipette 21a that can move to a position P3 in the upper direction of the measurement sample container 5 set on the rotary table 18a. When the measurement sample container 5 is transported by the rotary table 18a and arrives at the position P3, the sample suctioning unit 21 can suction the measurement sample in the measurement sample container 5. The sample suctioning unit 21 is connected to a flow cell 43 of the main detecting unit 22 (see Fig. 3A) through a flow path not shown in the drawing so as to be able to feed the measurement sample suctioned by the pipette 21a into the flow cell 43 of the main detecting unit 22.

The main detecting unit 22 has the flow cytometer 40 that is configured to detect lights from the measurement sample (forward scattered light, side scattered light, side fluorescent light), and outputs signals based on these lights (optical signals) to a circuit provided in a subsequent stage. The flow cytometer 40 will be described later referring to Figs. 3A and 3B.

A container cleaning unit 23 cleans inside of the measurement sample container 5 after the measurement sample is fed to the main detecting unit 22 by the sample suctioning unit 21. The container cleaning unit 23 is configured to discharge a cleaning solution into the measurement sample container 5 retained in the retaining portion 18b of the rotary table 18a and thereby clean the inside of the measurement sample container 5. When the same measurement sample container 5 is used again later to measure a different sample, the cleaning treatment prevents contamination of the sample with any other sample left in the measurement sample container 5.

Fig. 3A shows the configuration of the flow cytometer 40 of the main detecting unit 22. As shown in Fig. 3, laser light emitted from a semiconductor laser 41 is condensed on the measurement sample flowing in the flow cell 43 by a lens system 42 including a plurality of lenses. The sample suctioned by the pipette 21a of the sample suctioning unit 21 is fed into the flow cell 43.

As shown in Fig. 3B, the lens system 42 includes a collimator lens 42a, a cylindrical lens system (combination of a plano-convex lens 42b and a biconvex lens 42c), a condenser lens system (combination of a condenser lens 42d and a condenser lens 42e). These lenses are arranged in the mentioned order from the semiconductor-laser-41 side (from the left side shown in Figs. 3A and 3B).

A condensing lens 44 condenses the forward scattered lights from the cells of the measurement sample on a scattered light detector including a photo diode 45. A condensing lens 46 for side lights condenses the side scattered light and the side fluorescent light from the cells to be measured and the nuclei of these cells and guides the condensed lights to a dichroic mirror 47. The dichroic mirror 47 makes the side scattered light be reflected on a photo multiplier (photo-electron multiplier) 48 and transmits the side fluorescent light therethrough toward a photo multiplier (photo-electron multiplier) 49. Thus, the side scattered light is condensed on the photo multiplier 48, while the side fluorescent light is condensed on the photo multiplier 49. On these lights are reflected the characteristics of the cells and nuclei of the cells in the measurement sample.

The photo diode 45 and the photo multipliers 48 and 49 respectively convert the received optical signals into electrical signals and then output a forward scattered light signal (FSC), a side scattered light signal (SSC), and a side fluorescent light signal (SFL). The signals thus outputted are amplified by an amplifier not shown in the drawing and then outputted to a controller 24 (see Fig. 2) of the measuring device 2. The controller 24 has functions to, for example; process the optical signals outputted from the photo diode 45 and the multipliers 48 and 49, control the operations of the main detecting unit 22 and the sub-detecting unit 13, control the operations of the structural components 2a, 10 to 12, 14 to 21, and 23 wherein the sample is prepared, and transmit and receive various information to and from the data processing device 3.

The controller 24 has a computing unit such as a microprocessor, and a storage including, for example, ROM and RAM. In the storage, there are stored various data and control programs for controlling the operations of the detecting units 22 and 13 and the structural components 2a, 10 to 12, 14 to 21, and 23 wherein the sample is prepared. The signals FSC, SSC, and SFL processed by the controller 24 of the measuring device 2 are transmitted to the data processing device 3 by way of a communication unit.

As described, the main detecting unit 22 has a flow cytometer 40 shown in Fig. 3A, and outputs the forward scattered light signal (FSC), side scattered light signal (SSC), and side fluorescent light signal (SFL) obtained from the measurement sample. The controller 24 executes necessary signal processes to the signals outputted from the main detecting unit 22.

The sub-detecting unit 13 has a flow cytometer 40 almost identical to a flow cytometer provided in the main detecting unit 22, description of which, therefore, is omitted. The sub-detecting unit 13 measures the concentration of the sample before the measuring operation performed by the main detecting unit 22 starts. According to the example, the sub-detecting unit 13 is configured to obtain the forward scattered light signal (FSC), and outputs a signal for counting the cells corresponding to the sizes of epithelial cells based on the obtained forward scattered light signals. The forward scattered light signal FSC outputted from the sub-detecting unit 13 is inputted to and processed by the controller 24.

### [Configuration of data processing device]

As shown in Fig. 1, the data processing device 3 includes a personal computer, for example, a laptop PC or a desktop PC, and has an input unit 31, a display unit 32, and a controller 33. The controller 33 has a CPU, a storage including ROM, RAM, and a hard disc, a reader to read data such as CD-ROM drive, and input and output interfaces. In the storage of the controller 33, there are installed programs such as operating system and application programs, and operation programs for transmission of operation commands to the controller 24 of the measuring device 2 shown in Fig. 2, reception and analysis of results of the measuring operation performed by the measuring device 2, and display of results of the analysis. The data processing device 3 is connected to the controller 24 of the measuring device 2 to allow communication therebetween so that data is transmitted and received to and from the measuring device 2 and the data processing device 3.

By executing the programs installed in the storage, the CPU of the data processing device 3 obtains characteristic parameters such as intensities of the forward scattered light and side fluorescent light based on the signals FSC, SSC, and SFL. The CPU then creates frequency distribution data used to analyze the cells and cell nuclei based on the obtained characteristic parameters. Then, the CPU of the data processing device 3 executes a discriminating process to particles in the measurement sample based on the frequency distribution data to determine canceration of the cells.

### [Steps of analysis by cell analyzer 1]

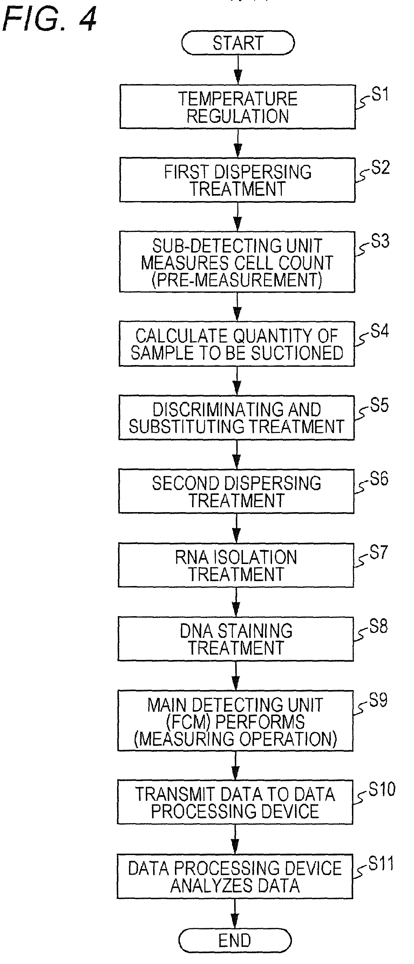

To analyze the cells using the cell analyzer 1, an operator of the cell analyzer sets the sample container 4 containing the biosample (cells) and the medium consisting primarily of methanol in the analyte setting unit 2a (see Fig. 2) to start the analysis by the cell analyzer 1. Hereinafter, steps of the analysis by the cell analyzer 1 are described referring to Fig. 4.

When the measuring operation starts, the temperature regulating unit 10 regulates the temperature of the medium first of all. Specifically, the sample in the sample container 4 set in the analyte setting unit 2a is suctioned by the analyte pipette unit 11 and fed into the sample container set in the sample setting portion 10a. Then, the temperature of the medium in the sample container is regulated to a predetermined degree (Step S1). The temperature is regulated by the temperature regulating unit 10 to a temperature almost equal to or higher than the room air temperature of a room where the cell analyzer 1 is installed (temperature of installation environment). The room air temperature of a room where the cell analyzer 1 is installed is normally approximately 22°C to 25°C (at most approximately 30°C). The temperature regulating unit 10 according to the example is configured to warm the medium until the temperature thereof increases to approximately 37°C. The temperature regulating unit 10 is configured to warm the medium for 10 minutes. Herein, the preservation temperature of the medium, though regulated to control the temperature-dependent variability of optical information, is not strictly limited as far as it stays in the range of 25°C to 50°C. To more effectively control the temperature-dependent variability of optical information, the preservation temperature of the medium is preferably regulated to 30°C to 45°C, more preferably 35°C to 45°C, and even more preferably 35°C to 40°C.

When the temperature of the medium is thus regulated by the temperature regulating unit 10, the temperature of the medium can be kept at substantially constant degrees irrespective of the preservation temperature of the medium before being fed to the cell analyzer 1. This accelerates alcohol fixation of the cells in the medium, thereby stabilizing the cells. Such a technical advantage can effectively control the temperature-dependent variability of optical signals (optical information) measured later by the main detecting unit 22 and the sub-detecting unit 13. We conducted the tests to verify variability of the optical signals is caused by changes of the preservation temperature of the medium, and such variability of the optical signals could be controlled by regulating the temperature of the medium. The details of the tests will be described in detail later. In order to effectively control variability of the optical signals, results of these tests were used to optimize a range of degrees and a length of time to be set for the temperature regulation of the medium by the temperature regulating unit 10 according to the example.

The biosample preserved in the medium temperature-regulated by the temperature regulating unit 10 is then subjected to the dispersing treatment performed by the first dispersing unit 12 (first dispersing treatment) to disperse aggregated cells in the sample (Step S2). Specifically, the sample in the sample container set in the sample setting portion 10a of the temperature regulating unit 10 is suctioned by the analyte pipette unit 11 and fed into the sample container portion 12a. Then, the sample in the sample container portion 12a is dispersed by the first dispersing unit 12.

When the first dispersing treatment is over, the analyte pipette unit 11 feeds the dispersion-completed sample into the sample fetching portion 13a of the sub-detecting unit 13, and a predetermined quantity of the dispersion-completed sample is flown into the flow cell of the sub-detecting unit 13 similar to the flow cell 43 shown in Fig. 3A. The sub-detecting unit 13 detects the cell count of epithelial cells in the sample (pre-measurement) by flow cytometry (Step S3). According to the example, the concentration of the sample is calculated based on the cell count of epithelial cells and the volume of the sample fed to the sub-detecting unit 13.

Next, the controller 24 decides a quantity of sample to be suctioned based on the calculated concentration to prepare the measurement sample used in the measuring operation (Step S4). That is, a quantity of sample by liquid measure necessary for the measuring operation is calculated based on the concentration of the sample used in the pre-measurement (cell count per unit volume) and the cell count of epithelial cells necessary for detecting any cancerous cells in the measuring operation. According to the example, the cell count of epithelial cells to be fed into the flow cell 43 of the main detecting unit 22 is a predetermined cell count (A). In this case, it is necessary for the cell count of epithelial cells in the sample fed to the discriminating and substituting unit 14 to be a larger cell count (B) than the predetermined cell count (A). Therefore, the quantity of sample by liquid measure is calculated in Step S4 so that the cell count (B), which is larger than the predetermined cell count (A), of epithelial cells are fed to the discriminating and substituting unit 14.

Next, the sample in the calculated quantity by liquid measure is subjected to a discriminating and substituting treatment (Step S5). That is, the analyte pipette unit 11 is driven by the controller 24 to suction the sample after the first dispersing treatment is performed thereto by the first dispersing unit 12. The analyte pipette unit 11 suctions the dispersion-completed sample in the exact quantity calculated earlier from the sample container portion 12a of the first dispersing unit 12. The suctioned sample is fed to the discriminating and substituting unit 14, in response to which the discriminating and substituting treatment starts.

Next, the aggregated cells in the sample are dispersed by the second dispersing unit 16 (second dispersing treatment) (Step S6). Specifically, the container transport unit 15 holds and removes the measurement sample container 5 from the retaining portion 18b of the reaction unit 18, and then locates the measurement sample container 5 in the sample delivering and receiving portion 11b. Then, the sample suctioned from the discriminating and substituting unit 14 by the analyte pipette unit 11 is fed into the measurement sample container 5 located in the sample delivering and receiving portion 11b. Then, the measurement sample container 5 containing the sample is transported by the container transport unit 15 to the second dispersing unit 16, and the sample therein is subjected to the second dispersing treatment.

When the second dispersing treatment is over, the measurement sample container 5 containing the dispersion-completed sample is set in the retaining portion 18b of the reaction unit 18. Then, the reagent (including RNase, hereinafter referred to as a RNA isolating agent) is added to the sample by the first reagent adding unit 19. Then, the measurement sample container 5 is warmed by the reaction unit 18, and the cells to be measured in the measurement sample container 5 are subjected to the RNA isolation treatment (Step S7). After the RNA isolation treatment is performed, the reagent (staining solution) is added to the sample by the second reagent adding unit 20. Then, the measurement sample container 5 is warmed by the reaction unit 18, and the cells to be measured in the measurement sample container 5 are subjected to the DNA staining (Step S8). According to the present embodiment, the cell count of significant cells necessary for the measuring operation is substantially constant because of the pre-measurement. When the cells are stained, therefore, a staining degree is less variable from one measuring operation to another. According to the present embodiment, the temperature of the medium is regulated to be substantially constant in the temperature regulating step (Step S1). This accelerates degeneration of chromatin included in the cells of the medium, thereby stabilizing the cells. As a result, the staining degree becomes less variable.

Next, the DNA-stained measurement sample is suctioned by the sample suctioning unit 21. The suctioned measurement sample is transferred to the flow cell 43 of the main detecting unit 22 (see Fig. 3A) to perform the measuring operation to the cells in the measurement sample (Step S9).

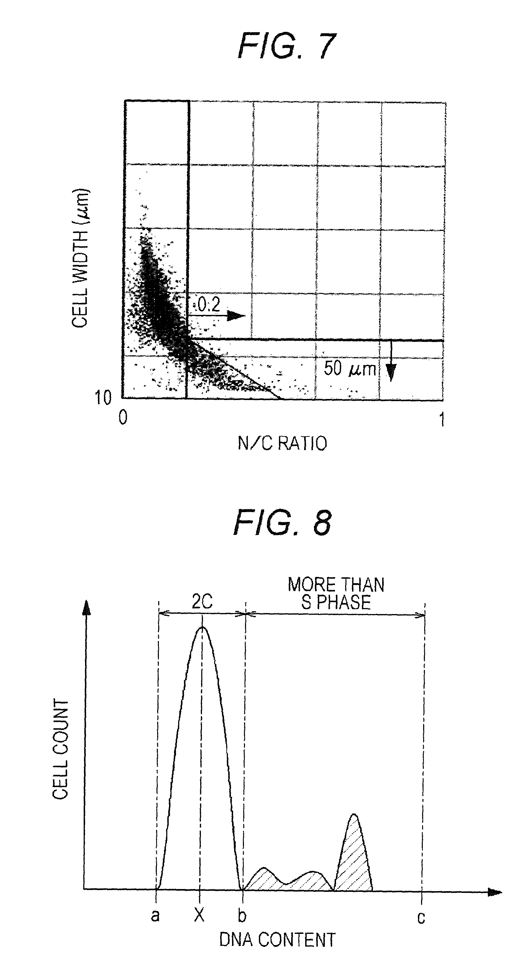

When the measuring operation is over, measured data thereby obtained is transmitted from the controller 24 of the measuring device 2 to the data processing device 3 (Step S10). Specifically, the forward scattered light signal (FSC), side scattered light signal (SSC), and side fluorescent light signal (SFL) obtained from each cell of the measurement sample are transmitted to the data processing device 3. The controller 33 (CPU) of the data processing device 3 constantly detects whether the measured data is received from the measuring device 2. When the measured data is received from the measuring device 2, the controller 33 of the data processing device 3 executes the analysis based on the measured data (Step S11). The details of the analysis in Step S11 will be described later referring to Figs. 7 and 8.

### [Steps of obtaining canceration information]

Next, steps of obtaining canceration information according to the present embodiment are described.

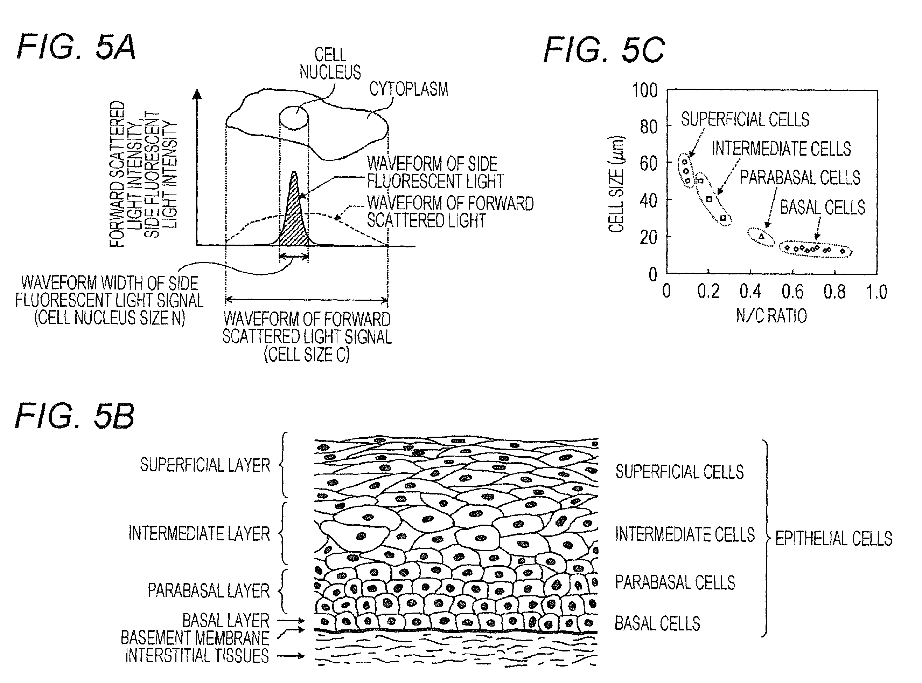

Fig. 5A is a diagram of the forward scattered light signal (FSC) and side fluorescent light signal (SFL) obtained in the measuring operation (Step S9 in Fig. 4). Fig. 5A schematically shows a cell including cell nucleus and waveforms of a forward scattered light signal and a side fluorescent light signal obtained from the cell. The longitudinal axis represents intensities of the lights. The waveform width of the forward scattered light intensity has a value representing cell width (cell size C). The waveform width of the side fluorescent light intensity represents a value representing cell nucleus width (cell nucleus size N). As shown with a shaded part, an area dimension of a region defined by the waveform of the side fluorescent light intensity and a predetermined base line represents the DNA content of the cell.

Fig. 5B is a schematic enlarged view in cross section of epithelial cells in uterine cervix. As shown in Fig. 5B, uterine cervix has layers, from the basement-membrane side; a layer formed by basal cells (basal layer), a layer formed by parabasal cells (parabasal layer), a layer formed by intermediate cells (intermediate layer), and a layer formed by superficial cells (superficial layer). The basal cells near the basement membrane are differentiated into the parabasal cells, the parabasal cells are differentiated into the intermediate cells, and the intermediate cells are differentiated into the superficial cells.

In the course of cancer development, the basal cells acquire dysplasia, transforming into dysplastic cells. The dysplastic cells acquire proliferation potency, occupying a region from the basal-layer side to the superficial-layer side. In epithelial cells in uterine cervix, therefore, cancerous cells are present in large numbers among the cells of the basal layer, parabasal layer, and intermediate layer in an early stage of cancer development. In an early stage of cancer development, however, there are a significantly smaller number of cancerous cells on the superficial-layer side of epithelial cells in uterine cervix.

It is known that the cell size of epithelial cells becomes smaller but the cell nucleus size thereof becomes larger from the superficial layer toward the layers on the basement-membrane side. Therefore, a ratio of the cell nucleus size (N) to the cell size (C) (hereinafter referred to as "N/C ratio") becomes accordingly larger from the superficial layer toward the layers on the basement-membrane side. The N/C ratio and the cell size C, therefore, has such a relationship as shown in Fig. 5C. Herein, the epithelial cells in uterine cervix that can be collected from test subjects are the cells of the parabasal layer, intermediate layer, and superficial layer. As described above, precancerous lesion develops in the cells on the basal-layer side in an early stage.

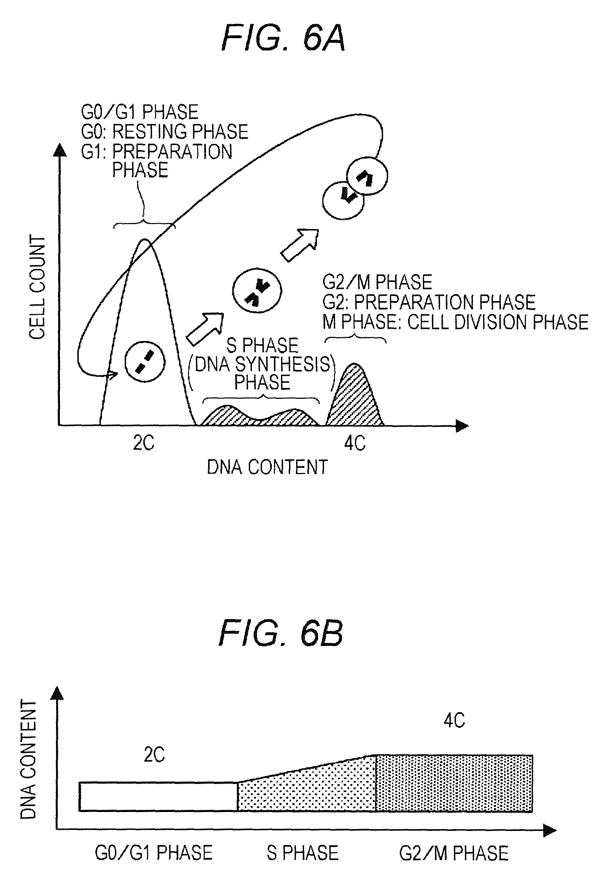

Fig. 6A is a diagram showing a relationship between cell count and DNA content in a cell cycle. As shown in Fig. 6A, a cell, in accordance with a certain cycle (cell cycle), is divided into two cells through events such as DNA duplication, chromosome partitioning, fission of cell nucleus, and cytoplasmic fission, and then back to where it started. The cell cycle can be divided into four phases; G1 phase (phase of preparation and check to enter S phase), S phase (phase of DNA synthesis), G2 phase (phase of preparation and check to enter M phase), and M phase (phase of cell division). Along with these four cycles, there is G0 phase where cell proliferation is stopped (resting phase), and thus there are five phases in total. Depending on the stage of a cell, the cell is in any of these phases.

When a cell is proliferating through the cell cycle, the nuclear chromosome of the cell increases as well. Therefore, the current phase of the cell in the cell cycle can be estimated by measuring the DNA content of the cell. Describing the DNA content of a normal cell referring to Fig. 6B, the DNA content has a constant value (2C) in G0/G1 phase. In the following S phase, the DNA content gradually increases. In the following G2 phase, the DNA content has a constant value (4C), and the value remains unchanged in M phase. Herein, the value C represents genomic DNA content per haploid. That is, 2C represents the DNA content twice as large as the genomic DNA content per haploid, and 4C represents the DNA content four times as large as the genomic DNA content per haploid. The DNA content of a normal cell in G0 phase or G1 phase of the cell cycle is 2C. A histogram of the DNA content of normal cells was drawn, as shown in Fig. 6A. The highest peak in the histogram represents a cell in G0/G1 phase with a smallest DNA content. The second highest peak represents a cell in G2/M phase with a largest DNA content. An interval between these peaks represents cells in S phase.

In normal cells, the cell count of cells in S phase and G2/M phase is significantly smaller than the cell count of cells in G0/G1 phase. In cancerous cells, however, the cell count of cells in S phase and G2/M phase is larger than that of normal cells. In addition, chromosome number increases in cancerous cells, which increases the DNA content as well.

The present embodiment, therefore, employs a screening method that focuses on the N/C ratio and the DNA content to determine whether the cells are cancerous.

Specifically, the screening method is to extract any cells with large values of the N/C ratio. Then, of the extracted cells, the cells with large DNA content and the cells with small DNA content are identified, so that the cells more likely to be cancerous (first cells) and the cells less likely to be cancerous (second cells) are effectively extracted. Generally, the first cells increase while the second cells decrease with the growth of cancerous tissues. Comparing cancerous tissues to normal tissues, a ratio of the cell count of first and second cells largely differs. Therefore, canceration is determined based on the ratio. Using the ratio of two different types of cells that increase and decrease in reverse proportion to each other, highly reliable screening outcomes can be obtained even from measurement samples with fewer target cells.

Specifically, the controller 33 of the data processing device 3 draws a scattergram indicating a relationship between the N/C ratio and the cell size (cell width) as shown in Fig. 7. Then, the controller 33 extracts measured data as data to be analyzed of any cell whose cell size is within a predetermined range (for example, equal to or larger than 10 µm and equal to or smaller than 50 µm) and whose N/C ratio is within a predetermined range (for example, equal to or larger than 0.2 and equal to or smaller than 1). This excludes the superficial cells present in the superficial layer with a smaller number of cancerous cells from the data to be analyzed. Herein, processes may be performed before extracting the cells; for example, process to split the measured data of white blood cells and epithelial cells, or process to split the measured data of single epithelial cells and aggregated epithelial cells. The cell size may be disregarded, and the range of N/C ratio alone may be used to extract the measured data of the cells.

Next, the controller 33 of the data processing device 3 draws a histogram shown in Fig. 8 based on the extracted measured data of the cells. In the histogram, the lateral axis represents the DNA content, and the longitudinal axis represents the cell count.

The controller 33 of the data processing device 3 obtains in the histogram of Fig. 8 a cell count N1 of the cells whose DNA content is larger than that of normal cells in S phase (cell count of cells whose DNA content is larger than "b" and equal to or smaller than "c"). The cell count N1 is, in other words, the cell count of cells whose DNA content exceeds the DNA content of normal cells in G0 phase or G1 phase of the cell cycle as shown in Fig. 6A. The controller 33 further obtains a cell count N2 of cells whose DNA content is equal to 2C of normal cells (cell count of cells whose DNA content is equal to or larger than "a" and equal to or smaller than "b"). The cell count N2 is, in other words, the cell count of cells whose DNA content is equal to that of normal cells in G0 phase or G1 phase of the cell cycle.

Then, the controller 33 of the data processing device 3 obtains a value calculated by dividing the cell count N1 of cells whose DNA content is equal to or larger than that of normal cells in S phase by the cell count N2 of cells whose DNA content is 2C (ratio of the cell counts N1 and N2 (N1/N2). Further, the controller 33 determines whether the cells are cancerous by comparing the ratio of the cell counts N1 and N2 to a predetermined threshold. The criteria of canceration is; the N1/N2 value equal to or larger than the predetermined threshold is determined as positive, whereas the N1/N2 value smaller than the predetermined threshold is determined as negative.

Then, the controller 33 of the data processing device 3 displays the obtained canceration information on the display unit 32 or outputs the information to an external device. For example, when the value is determined as positive, an indication that reexamination is required is displayed on the display unit 32. When the value is determined as negative, an indication that reexamination is not required is displayed on the display unit 32.

### [Conditions of temperature regulation by temperature regulating unit 10]

As described above, the temperature regulating unit 10 of the measuring device 2 regulates the temperature of the medium to a predetermined degree before the biosample is transported to the main detecting unit 22 or the sub-detecting unit 13. The optical signals obtained by the main detecting unit 22 and the sub-detecting unit 13 may become variable in the case where the temperature of the medium changes, and as a result, the variability involves the risk of inaccuracy in determining canceration based on the optical signals. For example, variability of the side fluorescent light signal and forward scattered light signal leads to variability of the cell nucleus size N and the cell size C, possibly adversely affecting the process of drawing the scattergram shown in Fig. 7. Further, variability of the side fluorescent light signal leads to variability of the DNA content of cells. This leads to the failure to accurately draw the histogram shown in Fig. 8, possibly adversely affecting the determination of canceration. Especially, the intensity of the side fluorescent light signal changes depending on a DNA staining degree. In view of the fact, non-uniform temperatures of the medium are likely to adversely affect the DNA staining degree.

The inventors of the present invention conducted the tests hereinafter described and thereby verified that variability of the optical signals could be controlled by regulating the temperature of the medium containing the biosample to a predetermined range of temperature degrees. The inventors also obtained regulation temperature and regulation time that effectively controlled variability of the optical signals. Further, the inventors verified effects of the temperature regulation when an alcohol concentration of the medium was changed. Hereinafter, the tests will be described in detail. The tests 1 and 2 described below were conducted on epithelial cells in uterine cervix. The test 3 was conducted on epithelial cells in oral mucosa (hereinafter referred to as "oral cells").

### Test 1

The protocol of the test 1 is described below.

### 1. Materials

(Biosample) As the biosample, cultured cells were used; Hela cells obtained from the American Type Culture Collection (ATCC).

(Staining solution) As the staining solution, a solution was used in which PI (propidium iodide) supplied by Sigma was diluted with ethylene glycol.

(RNA isolating agent) As the RNA isolating agent, a solution was used in which RNaseA supplied by Sigma was diluted with a Tris hydraulic acid solution (pH 7.5, 10 mM).

(Medium) Four different media were prepared and used in accordance with the target cells to be tested;
medium 1: aqueous solution containing methanol by 40 vol% and acetic acid by 0.8 vol%,
medium 2: aqueous solution containing ethanol by 40 vol% and acetic acid by 0.8 vol%,
medium 3: aqueous solution containing methanol by 25 to 80 vol% and acetic acid by 0.8 vol%, and
medium 4: aqueous solution containing ethanol by 25 to 80 vol% and acetic acid by 0.8 vol%.

(Diluent) The Tris hydraulic acid solution (pH 7.5, 10 mM) was used.

### 2. Conditions

(Conditions of preservation) The media, in which the cultured cells were preserved, were left at rest at 4°C or 30°C for 24 hours. In clinical practice, media containing cells are normally preserved at 4°C to 30°C until tests are conducted. The media used in our tests were left at rest at the lowest degree (4°C) or the highest degree (30°C) in a normally employed range of preservation temperatures.

(Conditions of temperature regulation) After the media were left at rest as described, the temperatures of the media were regulated in three different patterns described below;
- pattern 1: warmed for 10 minutes at one of regulation temperature degrees selected from the range of 30°C to 50°C,
- pattern 2: warmed at the regulation temperature of 37°C for 10 minutes, and
- pattern 3: warmed at the regulation temperature of 37°C for one of lengths of time selected from the range of 3 to 20 minutes.

### 3. Test procedures

The cultured cells preserved in the media for 24 hours were warmed at the regulating temperatures in a microtube and subjected to centrifugal separation (10000 rpm, 1 min.). Then, supernatant was removed from the media by an aspirator. After that, 1 ml of the diluent was added to the residual solutions (30 µL). Then, the media were again subjected to centrifugal separation (10000 rpm, 1 min.), and supernatant was removed from the media by the aspirator. The staining solution, RNA isolating agent, and diluent were added to the residual solutions (30 µL) to prepare measurement samples. Then, the prepared measurement samples were left at rest under the conditions of temperature regulation (any of the patterns 1 to 3). After that, the measurement samples were flown into a flow cytometer to obtain the optical signals (side fluorescent light signal, forward scattered light signal). Then, the optical signals obtained from the media respectively preserved at 4°C and 30°C were compared to determine any variability of the optical signals.

### Test results

### (Test 1-1)

This test was conducted on determining variability of an amount of fluorescence between the medium 1 (methanol-based solution) containing the cells and temperature-regulated to the range of 30°C to 50°C and the same medium whose temperature was not regulated (at 4°C or 30°C without warming). The amount of fluorescence is represented by an area dimension of the waveform of the side fluorescent light signal. There is correlation between the amount of fluorescence and the DNA content of the cell: the amount of fluorescence increases as the DNA content increases. This test and other tests hereinafter described obtained variability of the amount of fluorescence (value X) when 2C in Fig. 8 showed a peak.

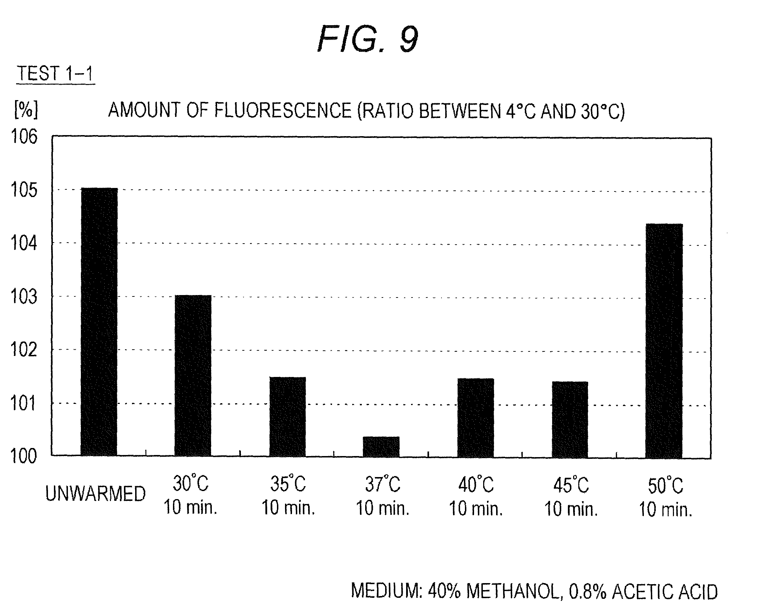

As shown in Fig. 9, the result of this test with the unwarmed medium showed the ratios of approximately 105% between the amount of fluorescence obtained from the medium preserved at 4°C and the amount of fluorescence obtained from the medium preserved at 30°C (ratio (percentage) was calculated by dividing a larger amount of fluorescence by a smaller amount of fluorescence). According to the result, the amount of fluorescence becomes more variable as the ratio increases.

Meanwhile, the temperature of the medium was regulated for 10 minutes to six different degrees (30°C, 35°C, 37°C, 40°C, 45°C, and 50°C) selected from the range of 30°C to 50°C. In all of these cases, the ratios between the amount of fluorescence obtained from the medium preserved at 4°C and the amount of fluorescence obtained from the medium preserved at 30°C were smaller than the result obtained with the unwarmed medium. This demonstrates that the temperature regulation to the range of 30°C to 50°C can reduce variability of the amount of fluorescence due to different preservation temperatures. The fluorescence ratio was particularly small in the range of 30°C to 45°C, and even smaller in the range of 35°C to 40°C. It was learned that, in the temperature regulation to 37°C, the fluorescence ratio was reduced to 101% or below, which was the best result. Based on the result, the preservation temperature in the temperature regulating unit 10 according to the present embodiment was set to 37°C. In the tests 1-2 to 2-3 hereinafter described, the preservation temperature was also set to 37°C which was expected to achieve the best result.

### (Test 1-2)

This test was conducted on variability of an amount of fluorescence between the medium 2 (ethanol-based solution) containing the cells and temperature-regulated to 37°C for 10 minutes and the same medium whose temperature was not regulated.

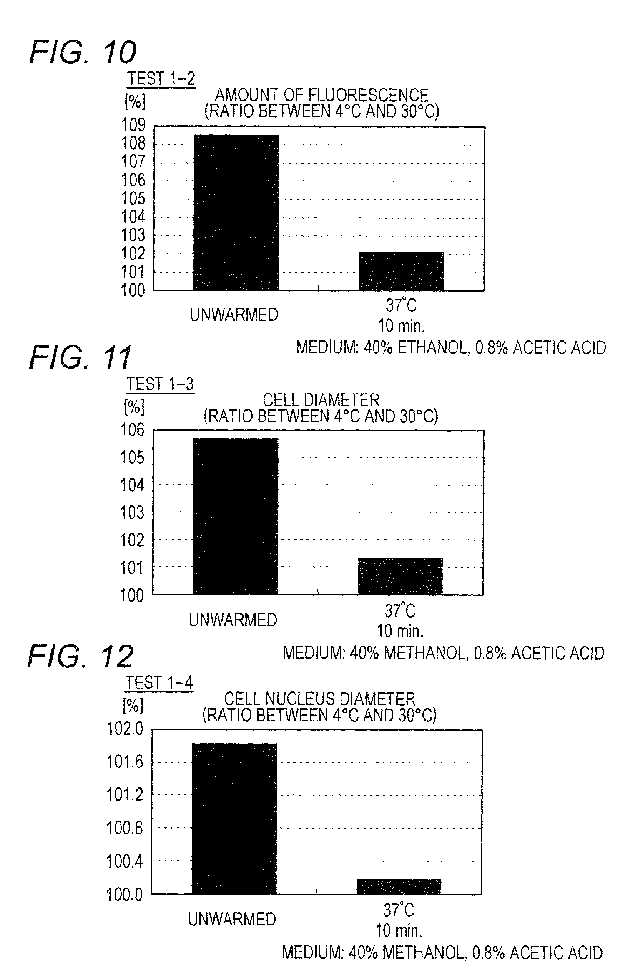

As shown in Fig. 10, the result of this test showed the ratios of approximately 102% between the amount of fluorescence obtained from the medium preserved at 4°C and the amount of fluorescence obtained from the medium preserved at 30°C. These ratios were smaller than the result with the unwarmed medium (108% to 109%). The temperature regulation was proven to reduce the fluorescence variability due to different preservation temperatures. The result of this test indicates that the temperature regulation is effective irrespective of the types of alcohols used in the medium as its base material.

### (Test 1-3)

This test was conducted on variability of a cell diameter (cell size C, see Fig. 5A) calculated from the forward scattered light signal between the medium 1 containing the cells and temperature-regulated to 37°C for 10 minutes and the same medium whose temperature was not regulated.

As shown in Fig. 11, the result of this test showed the ratios of approximately 102% between the cell diameter obtained from the medium preserved at 4°C and the cell diameter obtained from the medium preserved at 30°C. These ratios were smaller than the result with the unwarmed medium (105% to 106%). The result of this test demonstrates that the temperature regulation can reduce variability of the cell diameter due to different preservation temperatures.

### (Test 1-4)

This test was conducted on variability of a cell nucleus diameter (cell nucleus size N, see Fig. 5A) calculated from the side fluorescent light signal between the medium 1 containing the cells and temperature-regulated to 37°C for 10 minutes and the same medium whose temperature was not regulated.

As shown in Fig. 12, the result of this test showed the ratios of 100.0% to 100.4% between the cell nucleus diameter obtained from the medium preserved at 4°C and the cell nucleus diameter obtained from the medium preserved at 30°C. These ratios were smaller than the result with the unwarmed medium (101.6% to 102.0%). The temperature regulation was proven to reduce variability of the cell nucleus diameter due to different preservation temperatures.

It was learned from the results of the tests 1-3 and 1-4 that the temperature regulation of the medium could reduce variability of the N/C ratio.

### (Test 1-5)

This test was conducted on variability of an amount of fluorescence in the plural media 3 respectively containing methanol at different concentrations and temperature-regulated to 37°C for 10 minutes and the same media whose temperatures were not regulated.

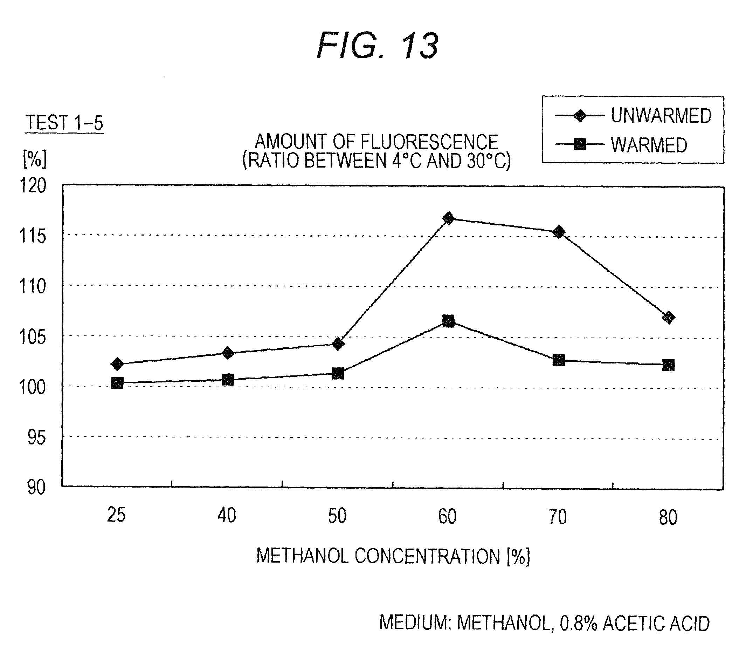

In any of the media irrespective of the methanol concentrations thereof, the result of this test confirmed that a ratio between the amount of fluorescence obtained from the medium preserved at 4°C and the amount of fluorescence obtained from the medium preserved at 30°C was smaller than the result with the unwarmed medium, as shown in Fig. 13. This test result indicates that the advantage of the temperature regulation can be obtained irrespective of the methanol concentrations.

### (Test 1-6)

This test was conducted on variability of an amount of fluorescence in the plural media 4 respectively containing ethanol at different concentrations and temperature-regulated to 37°C for 10 minutes and the same media whose temperatures were not regulated.

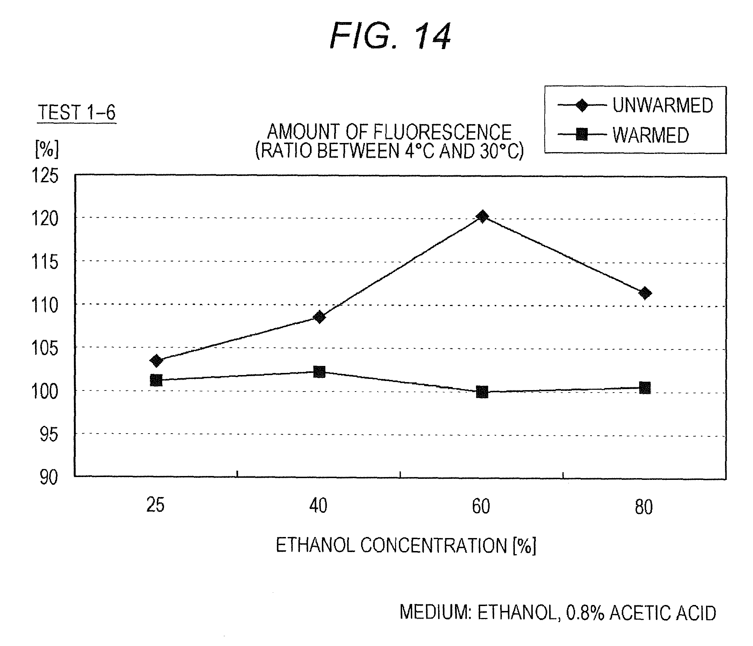

In any of the media containing ethanol at different concentrations, the result of this test confirmed that a ratio between the amount of fluorescence obtained from the medium preserved at 4°C and the amount of fluorescence obtained from the medium preserved at 30°C was smaller than the result with the unwarmed medium, as shown in Fig. 14. This result of this test indicates that the temperature regulation is very effective irrespective of ethanol concentrations.

### (Test 1-7)

This test was conducted on variability of an amount of fluorescence between the medium 1 containing the cells and temperature-regulated to 37°C for different lengths of temperature regulation time (3 to 20 minutes) and the same medium whose temperature was not regulated (temperature regulation time = 0 minute).

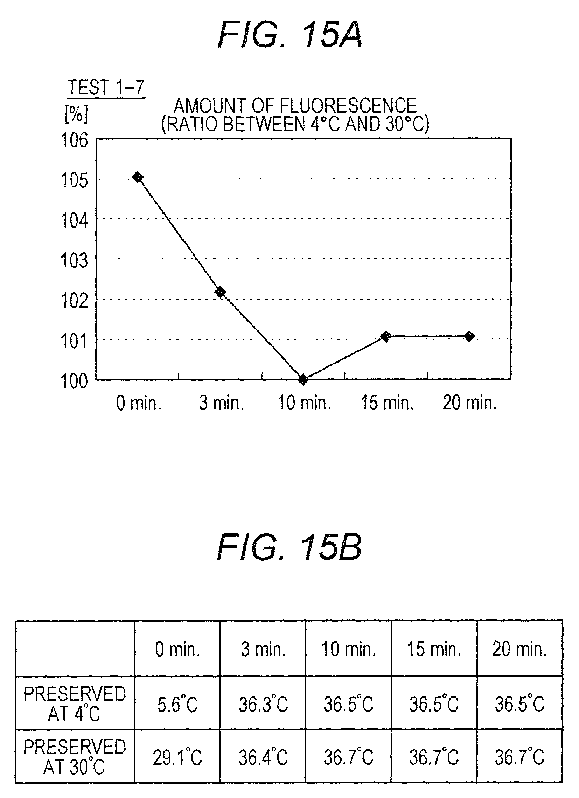

Irrespective of the length of temperature regulation time, the result of this test confirmed that a ratio between the amount of fluorescence obtained from the medium preserved at 4°C and the amount of fluorescence obtained from the medium preserved at 30°C was smaller than the result with the unwarmed medium, as shown in Fig. 15A. The temperature regulation was proven to reduce the fluorescence variability due to different preservation temperatures. Also, Fig. 15B shows temperature changes of the medium when regulated for different lengths of temperature regulation time. The drawing teaches that the temperature of the medium substantially increased to 37°C, which was the target regulation temperature, in three minutes after the temperature regulation started, and the fluorescence variability was accordingly reduced. The result of this test teaches that the fluorescence variability was minimized in 10 minutes after the temperature regulation started. Thus, based on the result, 10 minutes was set in the temperature regulating unit 10 according to the present embodiment as the medium temperature regulation time.

### Test 2

The protocol of the test 2 is described below.

### 1. Materials

(Biosample) As the biosample, clinical analytes including epithelial cells were collected from uterine cervixes of three patients with cervical cancer, respectively referred to as clinical analytes 1 to 3.

(Staining solution) The same staining solution as in the test 1 was used.

(RNA isolating agent) The same RNA isolating agent as in the test 1 was used.

(Medium) The same media as the medium 1 of the test 1 were used.

(Diluent) The same diluent as in the test 1 was used.

### 2. Conditions

(Conditions of preservation) The media, in which the clinical analytes were preserved, were left at rest at 4°C or 30°C for seven days.

(Conditions of temperature regulation) The media were thus left at rest and then warmed at the regulating temperature of 37°C for 10 minutes.

### 3. Test procedures

The samples were prepared in a manner similar to the test 1 by using the clinical analytes preserved in the media. The prepared samples were left at rest under the conditions of temperature regulation described earlier. After that, the measurement samples were respectively flown into a flow cytometer to obtain the optical signals (side fluorescent light signal, forward scattered light signal). Then, the optical signals obtained from the media respectively preserved at 4°C and 30°C were compared to determine any variability of the optical signals.

### Test results

This test was conducted on variability of an amount of fluorescence between the media respectively containing the clinical analytes 1 to 3 and temperature-regulated to 37°C for 10 minutes and the same media whose temperatures were not regulated.

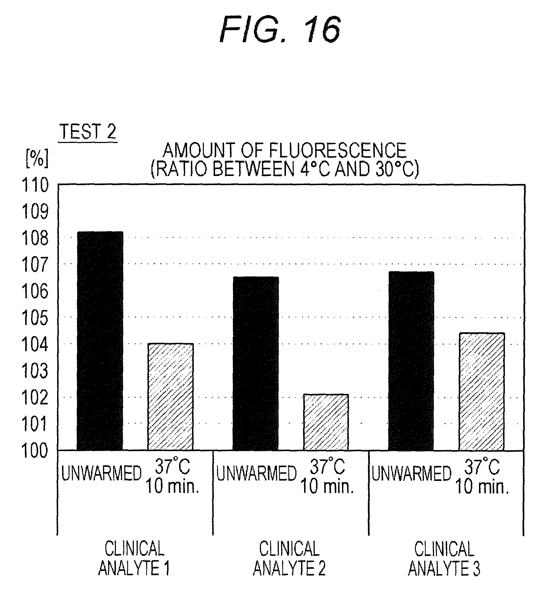

According to the result of this test, a ratio between the amount of fluorescence obtained from the medium preserved at 4°C and the amount of fluorescence obtained from the medium preserved at 30°C was 104.0% with the clinical analyte 1, 102.1% with the clinical analyte 2, and 104.4% with the clinical analyte 3 as shown in Fig. 16. These ratios were smaller than the result with the unwarmed media (108. 2%, 106.5%, and 106.7%). Thus, this test result demonstrates that the temperature regulation can reduce the fluorescence variability due to different preservation temperatures even with any of the clinical analytes collected from patients with cervical cancer.

### Test 3

The protocol of the test 3 on oral cells is described below.

### 1. Materials

(Biosample) The oral cells were collected in the following steps.

A swab was impregnated with an adequate quantity of saliva, and the swab was rubbed against the mouth cavity for 10 seconds (one each for right-cheek side and left-cheek side, two swabs in total were used). The swab rubbed against the mouth cavity was immediately put in 2.2 mL of the medium in a container having the capacity of 5.0 mL. The container was then rotated to wash the cells off the swab. Then, 440 µL of the medium was dispensed in each of microtubes having the capacity of 1.5 mL.

(Staining solution) The same staining solution as in the test 1 was used.

(RNA isolating agent) The same RNA isolating agent as in the test 1 was used.

(Medium) Aqueous solution containing methanol by 40 vol% and acetic acid by 0.8 vol%,

### 2. Conditions

(Conditions of preservation) The media, in which the oral cells were preserved, were left at rest at 4°C or 30°C for 24 hours.

(Conditions of temperature regulation) The media were thus left at rest and then warmed at the regulating temperature of 37°C for 10 minutes.

### 3. Test procedures

The measurement samples were prepared in a manner similar to those in the test 1, and the prepared samples were respectively flown into a flow cytometer to obtain optical signals (side fluorescent signal, forward scattered light signal). Then, the optical signals obtained from the media respectively preserved at 4°C and 30°C were compared to determine any variability of the optical signals.

### Test results

### (Test 3-1)

This test was conducted on variability of an amount of fluorescence between the medium containing the oral cells and temperature-regulated to 37°C for 10 minutes and the same medium whose temperature was not regulated.

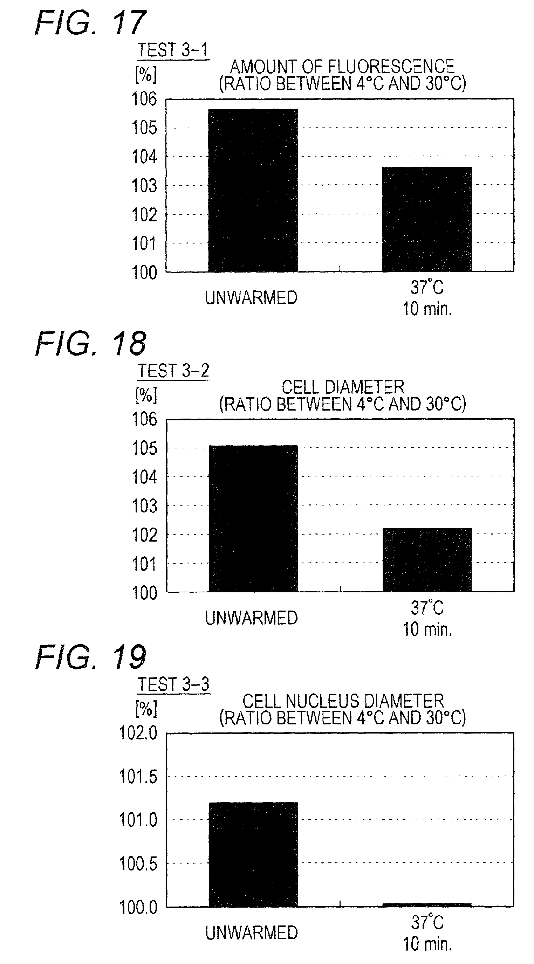

As shown in Fig. 17, the result of this test showed the ratios of 103% to 104% between the amount of fluorescence obtained from the medium preserved at 4°C and the amount of fluorescence obtained from the medium preserved at 30°C. These ratios were smaller than the result with the unwarmed medium (105% to 106%). Thus, the temperature regulation was proven to reduce the fluorescence variability due to different preservation temperatures.

### (Test 3-2)

This test was conducted on variability of a cell diameter (cell size C, see Fig. 5A) calculated from the forward scattered light signal between the medium containing the oral cells and temperature-regulated to 37°C for 10 minutes and the same medium whose temperature was not regulated.

As shown in Fig. 18, the result of this test showed the ratios of approximately 102% between the cell diameter obtained from the medium preserved at 4°C and the cell diameter obtained from the medium preserved 30°C. These ratios were smaller than the result with the unwarmed medium (105% to 106%). Thus, the temperature regulation was proven to reduce variability of the cell diameter due to different preservation temperatures.

### (Test 3-3)

This test was conducted on variability of a cell nucleus diameter (cell nucleus size N, see Fig. 5A) calculated from the side fluorescent light signal between the medium containing the oral cells and temperature-regulated to 37°C for 10 minutes and the same medium whose temperature was not regulated.

As shown in Fig. 19, the result of this test showed the ratios of 100.0% to 100.5% between the cell nucleus diameter obtained from the medium preserved at 4°C and the cell nucleus diameter obtained from the medium preserved at 30°C. These ratios were smaller than the result with the unwarmed medium (101.0% to 101.5%). Thus, the temperature regulation was proven to reduce variability of the cell nucleus diameter due to different preservation temperatures.

The result of the test 3 confirmed that variability of the optical signals due to different temperatures of the medium could be controlled with epithelial cells of oral mucosa as well as epithelial cells of uterine cervix when the temperature of the medium was regulated to predetermined degrees. As a result, information of the cells, such as DNA content, cell size, and cell nucleus size, can be accurately obtained.

The invention is not necessarily limited to the embodiment but may be suitably modified within the scope of the invention recited in the appended claims

While the cell analyzer is used to analyze epithelial cells in uterine cervix, the cell analyzer may be also used to, for example, analyze epithelial cells of oral mucosa as demonstrated by the test 3. The cell analyzer may be used to analyze epithelial cells of, for example, bladder, pharynx, or other organs.

According to the embodiment, the N/C ratio is calculated as the ratio between the value representing the cell nucleus width obtained based on the waveform width of the side fluorescent light intensity (cell nucleus size N) and the value representing the cell width obtained based on the waveform width of the forward scattered light intensity (cell size C). The N/C ratio is not necessarily limited thereto, and may be calculated as a ratio between cell nucleus area and cell area. According to the embodiment, the value representing the cell width was obtained based on the waveform width of the forward scattered light intensity (cell size C). This enables accurate measurement of cell size in the case where the cells flowing in the flow cell are elongated in a given direction.

As described so far, the medium according to the present embodiment is not particularly limited as far as the cells can be preserved therein. However, the medium contains alcohol. Though the alcohol included in the medium according to the present embodiment is not particularly limited, examples of the alcohol are methanol and ethanol. The alcohol concentration of the medium according to the present embodiment is not particularly limited as described in the tests 1-5 and 1-6. However, it is preferable that the alcohol concentration is equal to or larger than 25 vol% in terms of preservation of the cells because alcohol concentrations smaller than 25 vol% easily trigger cytoclasis. On the other hand, alcohol concentrations larger than 60% are likely to cause aggregation of the cells. Therefore, it is preferable that the alcohol concentration is equal to or smaller than 60 vol% when the cells are measured by flow cytometry using a flow cytometer. In summary, it is preferable that the alcohol concentration of the medium is equal to or larger than 25 vol% and equal to or smaller than 60 vol%.

According to the example, while the temperature regulating unit 10 is provided as a unit integral with the measuring device 2 of the cell analyzer 1, the temperature regulating unit 10 may also be provided separately from the measuring device 2 as an independent unit.

According to the embodiment, the optical information to be obtained from each cell of the measurement sample flowing in the flow cell is the forward scattered light signal (FSC), side scattered light signal (SSC), and side fluorescent light signal (SFL). However, the present invention is not necessarily limited thereto. According to the embodiment, for example, the side fluorescent light signal is used to obtain the value representing the DNA content of each cell. However, the optical signal used for the purpose is not necessary a side signal but may be a fluorescent light signal obtained at a different angle (for example, forward fluorescent light signal). The main detecting unit 22 may be further provided with an imaging unit, wherein an image signal of each cell in the measurement sample flowing in the flow cell is obtained as the optical information. In that case, the imaging unit has, for example, a light source including a pulse laser and a camera, wherein laser light emitted from the pulse laser enters the flow cell 43 through a lens system and further transmits through an object lens and a dichroic mirror, finally forming an image on the camera. The pulse laser emits light by a predetermined timing that enables the camera to capture an image.

## Claims

1. A method for analyzing a cell comprising:
regulating a temperature of a medium comprising alcohol, wherein the cell is fixed by the medium;
staining DNA of the alcohol-fixed cell with a fluorescent staining solution containing a dye;
flowing the cell into a flowcytometer; and
obtaining a fluorescent light information by irradiating light from a light source of the flowcytometer on the cell,
the method is **characterized in that** the temperature of the medium is regulated to 35°C to 45°C.

2. The method according to claim 1, further comprising:
preparing a measurement sample by substituting the medium with a diluent before the fluorescent light information obtaining step.

3. The method according to claim 1, further comprising:
obtaining a cell information based on the fluorescent light information,
wherein the cell information is at least one selected from the group consisting of DNA content and cell nucleus size.

4. The method according to claim 1, further comprising:
obtaining a scattered light information by irradiating light from a light source of the flowcytometer on the cell; and
obtaining a cell information based on the scattered light information,
wherein the cell information is an information on canceration of the cell.

## Patentansprüche

1. Verfahren zum Analysieren einer Zelle, welches umfasst:
Regulieren einer Temperatur eines Alkohol umfassenden Mediums, wobei die Zelle durch das Medium fixiert wird;
Färben von DNA der Alkohol-fixierten Zelle mit einer Fluoreszenzfärbungs-Lösung, enthaltend einen Farbstoff;
Leiten der Zelle in ein Durchflusszytometer; und
Gewinnen einer Fluoreszenzlicht-Information durch Bestrahlen der Zelle mit Licht aus einer Lichtquelle des Durchflusszytometers,
wobei das Verfahren **dadurch gekennzeichnet ist, dass** die Temperatur des Mediums auf 35°C bis 45°C reguliert wird.

2. Verfahren gemäß Anspruch 1, welches weiterhin umfasst:
Herstellen einer Messprobe durch Ersetzen des Mediums durch ein Verdünnungsmittel vor dem Schritt des Gewinnens der Fluoreszenzlicht-Information.

3. Verfahren gemäß Anspruch 1, welches weiterhin umfasst:
Gewinnen einer Zellinformation basierend auf der Fluoreszenzlicht-Information,
wobei die Zellinformation wenigstens eine, ausgewählt aus der Gruppe, bestehend aus DNA-Gehalt und Zellkerngröße, ist.

4. Verfahren gemäß Anspruch 1, welches weiterhin umfasst:
Gewinnen einer Streulicht-Information durch Bestrahlen der Zelle mit Licht aus einer Lichtquelle des Durchflusszytometers, und
Gewinnen einer Zellinformation basierend auf der Streulicht-Information,
wobei die Zellinformation eine Information über die Karzinogenese der Zelle ist.

## Revendications

1. Procédé d'analyse d'une cellule comprenant :
la régulation d'une température d'un milieu comprenant de l'alcool, dans lequel la cellule est fixée par le milieu ;
la coloration de l'ADN de la cellule fixée par l'alcool avec une solution de coloration fluorescente contenant un colorant ;
le passage de la cellule dans un cytomètre en flux ; et
l'obtention d'informations de lumière fluorescente par exposition de la cellule à une lumière d'une source de lumière du cytomètre en flux,
le procédé est **caractérisé en ce que** la température du milieu est régulée à 35 °C à 45 °C.

2. Procédé selon la revendication 1, comprenant en outre :
la préparation d'un échantillon de mesure par substitution du milieu avec un diluant avant l'étape d'obtention des informations de lumière fluorescente.

3. Procédé selon la revendication 1, comprenant en outre :
l'obtention d'informations relatives à la cellule sur la base des informations de lumière fluorescente,
dans lequel les informations relatives à la cellule sont au moins des informations sélectionnées dans le groupe constitué de la teneur en ADN et de la taille du noyau de la cellule.

4. Procédé selon la revendication 1, comprenant en outre :
l'obtention d'informations de lumière diffusée par exposition de la cellule à une lumière d'une source de lumière du cytomètre en flux ; et
l'obtention d'informations relatives à la cellule sur la base des informations de lumière diffusée,
dans lequel les informations relatives à la cellule sont des informations relatives à la cancérisation de la cellule.
